# EUROPEAN PATENT APPLICATION

(11) **EP 4 606 898 A1**
(43) Date of publication of application: **27.08.2025**
(21) Application number: 23880059.3
(22) Date of filing: 21.09.2023
(51) Int. Cl.: C12N 15/74, C07K 14/335, C12P 21/00

(54) **CELL SURFACE LARGE-DISPLAY TECHNOLOGY USING EXTRACELLULAR MEMBRANE LIPOPROTEIN PRSA DISPLAY SYSTEM**

(30) Priority: 17.10.2022 KR 20220133553
(71) Applicant: Future and Tech Co., Daejeon 34015 (KR)
(72) Inventor: KIM, Kwang, Daejeon 34008 (KR); YANG, Chan Woo, Daejeon 34007 (KR); CHOI, Hye Jin, Daejeon 34008 (KR); HONG, Seung Pyo, Daejeon 34008 (KR)
(74) Representative: Cabinet Chaillot
(86) International application number: PCT/KR2023/014408
(87) International publication number: WO 2024/085480

(57) **Abstract**

The present invention relates to: lactic acid bacteria extracellular membrane protein PrsA having a cell surface display anchor motif function; and a vector system for cell surface display of a target protein, the system comprising a sequence that encodes the PrsA and a strong promoter that is upstream of the PrsA gene.

## Description

### [Technical Field]

The present invention relates to a lipoprotein PrsA and its derivatives present in the cell outer membrane of lactic acid bacteria for cell surface expression, a PrsA promoter that induces overexpression of PrsA, and a cell surface expression vector that encodes PrsA.

### [Background Art]

The cell surface display system facilitates the expression of proteins or peptides on the cell surface, enabling their exposure to the extracellular environment. This approach allows proteins or peptides displayed on the cell surface to be freely accessible in extracellular space. The technique of displaying foreign proteins on cell surfaces holds significant importance in the fields of biological engineering and industrial applications, including vaccine development, whole cell biocatalysis, bio-adsorbents, and biosensors.

To express a protein on the surface of a cell, it is necessary to have a secretion signal on the sequence of the protein that facilitates the translocation of synthesized proteins across the cell membrane. The surface expression systems for target proteins, enzymes, peptides, and other molecules vary depending on the type of cell. In bacteria, proteins synthesized in the cytoplasm or near the cell membrane require a signal sequence to facilitate their insertion into the cell membrane or secretion to the extracellular environment. In the case of gram-negative bacteria, they must pass through the inner membrane and the cell membrane space and be inserted or attached to the outer membrane to exposure outside the membrane. Therefore, the extracellular membrane protein and the enzyme or toxin protein secreted to the outside of the cell require a secretion signal and a targeting signal that settles on the cell surface.

There are four types of anchor proteins which can anchor a foreign protein to the thick cell wall of Gram-positive bacteria such as lactic acid bacteria, which are (i) a transmembrane anchor, (ii) a lipoprotein anchor, (iii) an LPXTG anchor, and (iv) an LysM-repeat anchor (Boekhorst et al. Microbiology, 2006, 152:3175-3183; Michon et al. Microb Cell Fact. 2016, 15:70).

In transmembrane anchor proteins belonging to the general membrane protein types of Gram-positive and Gram-negative bacteria, 20 to 30 amino acids at the N-terminus or C-terminus, which are composed of hydrophobic amino acids, are inserted into the cell membrane in an alpha-helix structure to form an anchor, which has a limitation in that they cannot be displayed in large quantities because they significantly affect the rigidity of the membrane when being displayed in large quantities on the cell membrane. The second type, which is a lipoprotein anchor, is present on the cell surface by a mechanism in which the -SH group of a cysteine residue present immediately behind a secretion signal is covalently bonded to the carbon of the glycerin head of a lipid, which is a cell membrane component, and anchored to the cell membrane. Unlike the above-described transmembrane anchor that is inserted into the cell membrane, the lipoprotein anchor hangs on the lipid bilayer without affecting the rigidity of the membrane. Thus, it has the great advantage of being able to be displayed in large quantities on the cell surface.

Meanwhile, in most cases, such as Gram-positive and Gram-negative bacteria or yeast, the biggest obstacle to commercialization of vaccine development and biocatalyst development using microbial surface display system is the difficulty in displaying a target protein in large quantities on a cell surface. Particularly, over the past 50 years of molecular biology research, large-quantity expression systems have been developed by altering the nucleotide sequence of a promoter in an expression system or using an inducer that binds to a repressor, and microbial hosts that can stably express a target protein in cells have been developed by manipulating or deleting a specific gene in the chromosome of specific bacteria used as a vector. However, to date, it is safe to say that there is no artificial technology that can express one specific target protein per microorganism at more than 1% of the total protein amount, except for certain industrial strains including *E. coli.* In addition, it can be said that there is no system that can display a specific target protein in large quantities on the cell membrane, rather than in the cytoplasm, or in the outer membrane. In the commercialization of cell surface display technology of a biocatalyst for a bioconversion process, the most important key factors are the amount of an enzyme displayed on the surface and the stability of the enzyme. Particularly, in vaccine development, the amount of bacteria that can be administered into the body is limited due to side effects such as inflammation. Therefore, when the total amount of antigen that can be presented is limited compared to the amount of administered bacteria, the induced immune response may be insufficient for disease prevention, and thus the amount of a target antigen displayed on a cell surface may be a very important key factor.

Lactic acid bacteria have long been used worldwide in fermented foods containing lactic acid bacteria. They are recognized as Generally Recognized As Safe (GRAS) microorganisms. Accordingly, various studies are being conducted using GRAS microorganisms, which have low toxicity and side effects and can relatively minimize the possibility of safety issues.

Meanwhile, in Korean Unexamined Patent Application Publication No. 2019-0037481, "Method for displaying target protein at cell surface using cell anchoring motif from corynebacteria" is disclosed, and in Korean Unexamined Patent Application Publication No. 2002-0010428, "Novel cell wall anchor proteins from yeast, genes thereof and cell surface expression systems using the same" using four types of glycosyl phosphatidyl inositol (GPI)-anchored proteins, such as HpSED1, HpGAS1, HpTIP1 and HpCWP1, isolated from the methanol-assimilating yeast, *Hansenula polymorpha,* is disclosed, and in Korean Unexamined Patent Application Publication No. 2004-0032824, "Surface expression vector using polygamma-glutamate synthetic gene derived from strain in Bacillus (*Bacillus subtilis* var. chungkookjang), and method for expressing protein on surface of microorganism using the same" is disclosed. However, "Surface-display overexpression technique using extracellular membrane lipoprotein PrsA display system" using the lactic acid bacterial surface protein PrsA or its variant of the present invention, and a PrsA promoter has not been disclosed.

### [Disclosure]

### [Technical Problem]

The present invention was derived in response to the above-mentioned needs, and the present inventors purified and cultured lactic acid bacteria, which are GRAS microorganisms, from Korean traditional fermented food, gat-kimchi (Leaf Mustard kimchi), and selected PrsA, an outer membrane lipoprotein, which is constantly displayed in large quantities on the surface of the separated GRAS lactic acid bacteria, and its promoter through a proteolytic surfaceshaving method.

The protein structure of the selected PrsA was analyzed, a vector for cell surface display in which the coding sequences of a PrsA wild-type, PrsA serine-rich domain-deleted variant, a PrsA hinge region-deleted variant, or a PrsA hinge region and the serine-rich domain-deleted variant is operably linked under the regulation of a PrsA promoter was constructed, and the cell surface display of a reporter protein was assessed by cloning the reporter gene in the vector and transforming the clones in lactic acid bacteria. As a result, it was observed that the reporter protein was stably displayed on the cell surface in all lactic acid bacteria transformed with a vector containing the coding sequence of the PrsA protein or its variant, confirming that the PrsA protein of the present invention and its variant can serve as a surface display anchoring motif, and thus the present invention was completed.

### [Technical Solution]

To solve the above-described problems, one aspect of the present invention provides a recombinant vector for the cell surface display of a target protein, in which a polynucleotide encoding PrsA consisting of the amino acid sequence of SEQ ID No. 2 or its variant and a gene encoding a target protein are sequentially linked downstream of a PrsA promoter consisting of the nucleotide sequence of SEQ ID No. 3.

Another aspect of the present invention provides a microorganism transformed with the recombinant vector.

Still another aspect of the present invention provides a method of displaying a target protein on the surface of a microorganism, which includes transforming a microorganism with the recombinant vector.

Yet another aspect of the present invention provides a method of preparing a microorganism with a target protein displayed on its cell surface, which includes culturing a microorganism transformed with the recombinant vector to display a target protein on the cell surface; and recovering the microorganism with the target protein displayed on its cell surface.

Yet another aspect of the present invention provides a microorganism with a target protein displayed on its cell surface, prepared by the above-described method.

Yet another aspect of the present invention provides an injectable preparation, which includes the microorganism with the target protein displayed on its cell surface as an active ingredient.

Yet another aspect of the present invention provides an oral preparation, which includes the microorganism with the target protein displayed on its cell surface as an active ingredient.

Yet another aspect of the present invention provides a composition for inducing immunity in vertebrates other than humans, which includes the microorganism as an active ingredient.

Yet another aspect of the present invention provides a method of manufacturing a protein array, including immobilizing a microorganism, which is prepared by the method of the present invention and has a target protein displayed on its surface, on a substrate surface.

Yet another aspect of the present invention provides a method of inducing immunity in vertebrates, including administering a microorganism, which is prepared by the method of the present invention and has an antigen displayed on its surface, to a vertebrate.

### [Advantageous Effects]

The present invention utilizes a protein isolated from lactic acid bacteria, which are GRAS bacteria, and provides a method of stably displaying a foreign protein on the surface of lactic acid bacteria. Therefore, it is expected to have high industrial utility because it can be used for cell surface display of foreign proteins that were previously difficult to display stably or in large quantities on the cell surface.

In addition, when a ligand protein, receptor protein or enzyme protein involved in *in vivo* signaling of a normal cell having a self-protein is displayed using the display system of the present invention, it can be used as a therapeutic agent for metabolic diseases, and when a ligand or receptor of an immune cell is expressed, it can be used as an immunotherapeutic agent, ,and when a bacterial antigen is expressed, it can be used as a preventive or therapeutic vaccine. Therefore, the display system of the present invention can be effectively used in the pharmaceutical industry.

### [Description of Drawings]

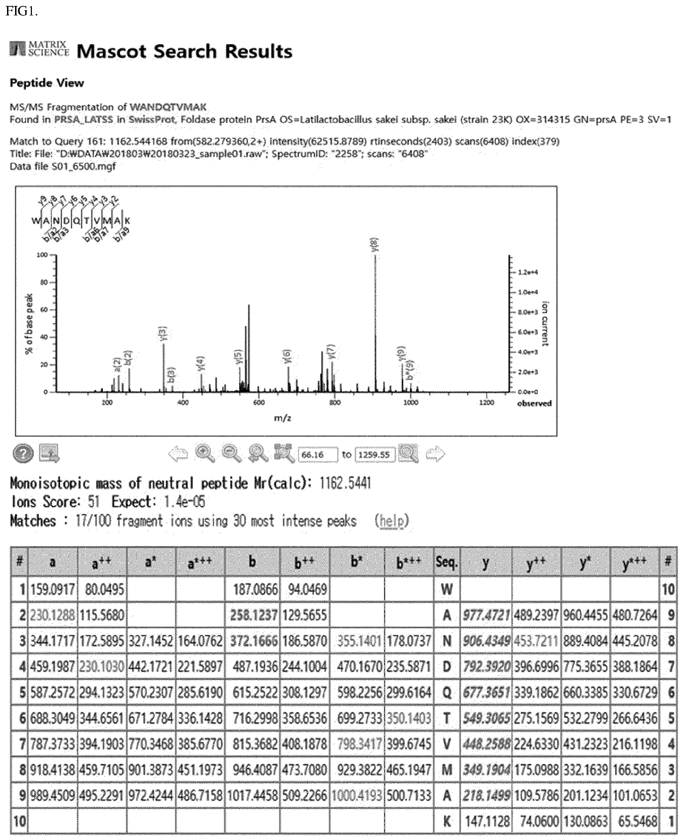

FIG. 1 shows the MS/MS results of the representative peptide sequence, amino acids 255 to 264 (SEQ ID No. 6), among the MS/MS analysis results of five Lys-C cleaved peptides previously confirmed.

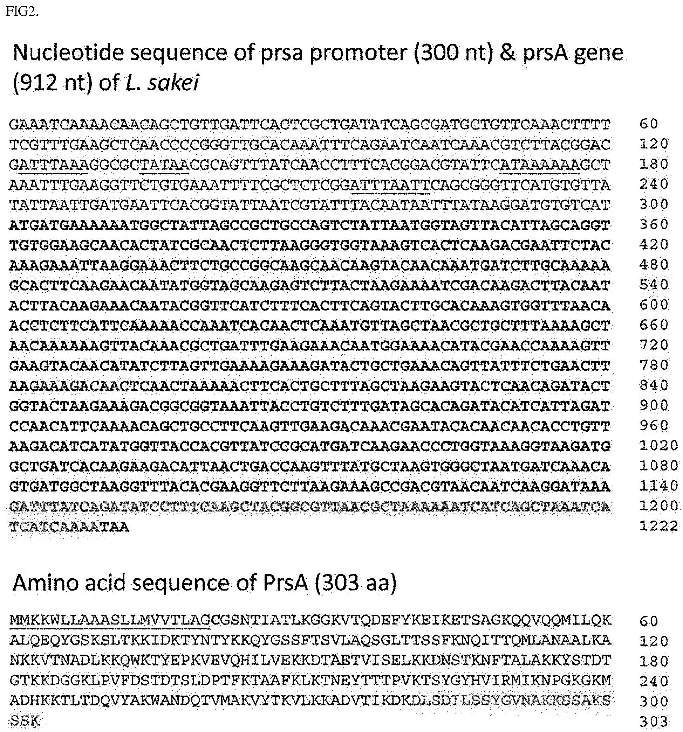

FIG. 2 shows the nucleotide sequence and amino acid sequence information of the PrsA protein of *Lactobacillus sakei.* In addition, a hinge region (dotted underline+bold text) and a serine-rich domain (solid underline+bold text), which are deleted in the preparation of a PrsA protein derivative, are shown.

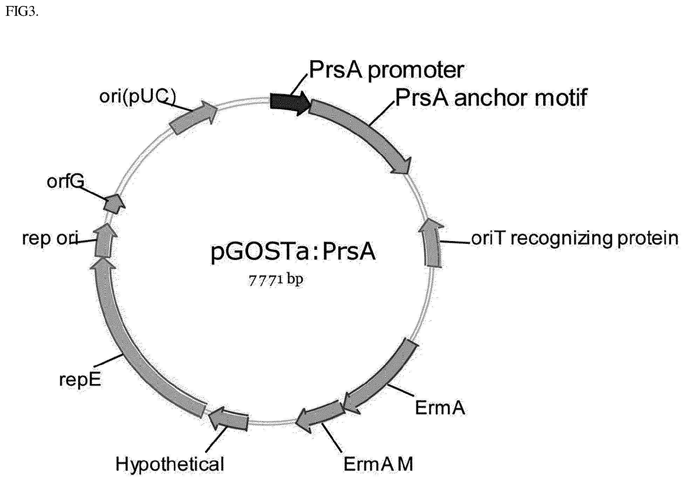

FIG. 3 is a map of the pGOSTalpha:PrsA vector containing the PrsA promoter sequence and the PrsA anchoring motif-coding sequence.

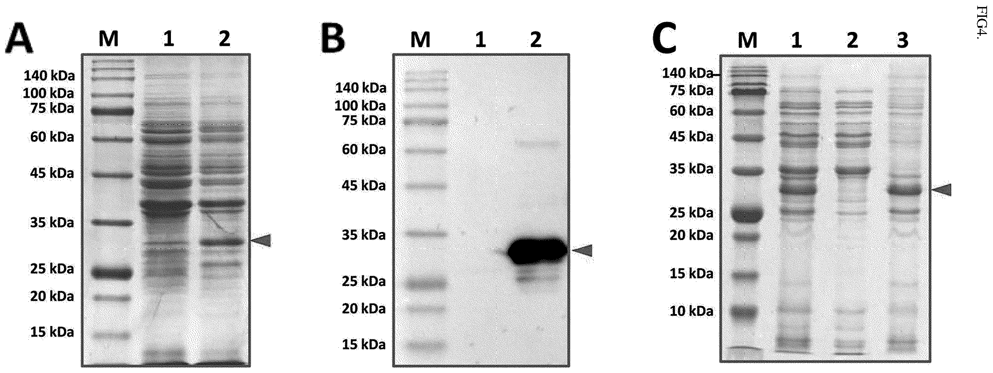

FIG. 4 shows the results of confirming the expression of *L. sakei-derived* PrsA in *Lactobacillus paracasei* (*L. paracasei*) transformed with the pGOSTα:PrsA vector: (A) is a CBB-stained gel image of the total protein; (B) is a Western blot result using an anti-PrsA antibody after transferring the total protein to a PVDF membrane; and (C) is the image of a gel showing fractions stained with CBB after the total protein is subjected to high-speed centrifugation to separate the cytoplasmic proteins and the membrane proteins. The location of the PrsA -anchor membrane protein (theoretical molecular weight, 31.4 kDa) is indicated by the red arrow. FIG. 4A and B: lane M: protein size marker (3-color Broad Range Protein Marker, #PM2700 Thermo), lane 1: non-transformed *L. paracasei,* lane 2: transformed *L*. *paracasei.* FIG. 4C: lane M: protein size marker, lane 1: total protein, lane 2: cytoplasmic protein fraction, lane 3: cell membrane protein fraction.

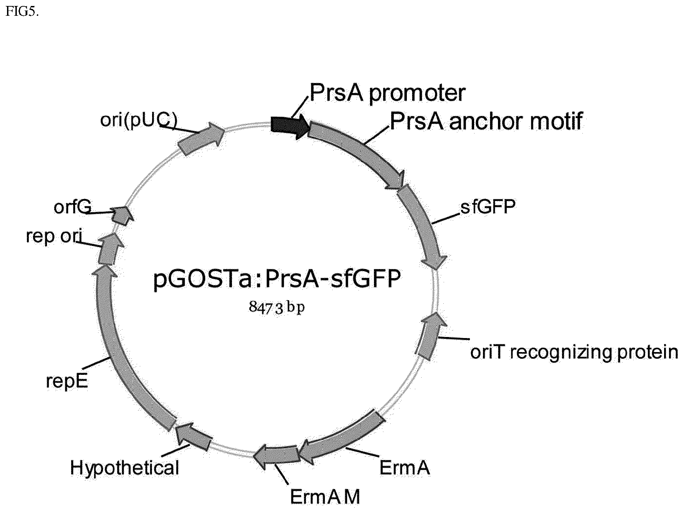

FIG. 5 is the vector map of pGOSTa:PrsA-sfGFP in which the reporter gene *sfGFP* is cloned into the vector of FIG. 3.

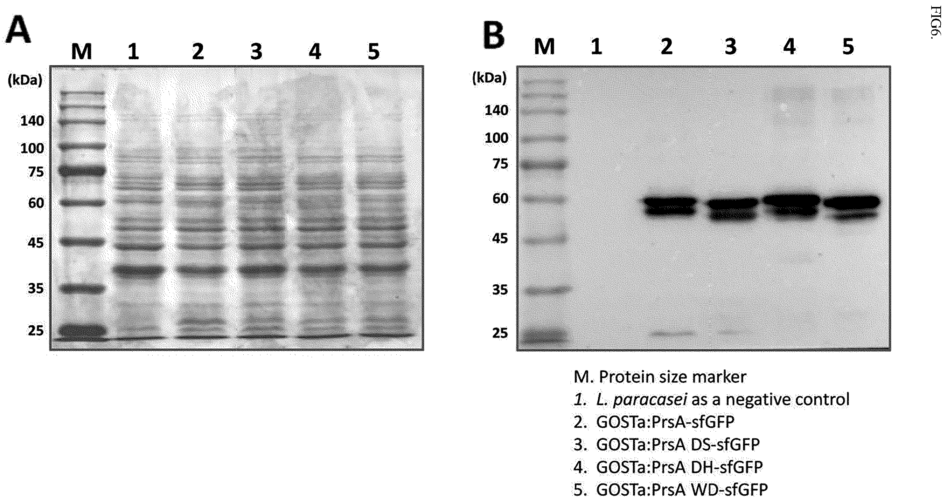

FIG. 6 shows the results of analyzing protein expression levels after transforming *L*. *paracasei* with recombinant vectors in which the sfGFP gene is fused to the 3' end of a PrsA, a PrsA serine-rich domain-deleted variant, a PrsA hinge region-deleted variant, and a PrsA serine-rich domain and hinge region-deleted variant gene (GOSTa:PrsA-sfGFP, GOSTa:PrsA DS-sfGFP, GOSTa:PrsA DH-sfGFP, and GOSTa:PrsA WD-sfGFP, respectively) and culturing the transformants: (A) is the image of a CBB-stained gel; and (B) is the Western blot result using an anti-GFP antibody.

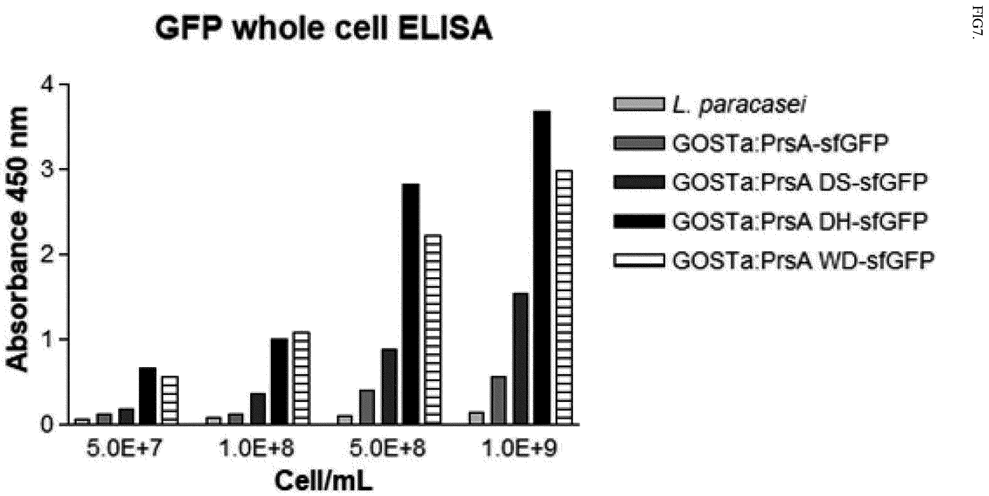

FIG. 7 shows the comparison of the amounts of sfGFP surface-displayed outside a cell membrane by analyzing the sfGFP displayed on the cell surface of lactic acid bacteria using anti-GFP antibody using whole-cell ELISA, after transforming *L. paracasei* with recombinant vectors in which the sfGFP gene is fused to the 3' end of a PrsA, PrsA serine-rich domain-deleted variant, PrsA hinge region-deleted variant and PrsA serine-rich domain and hinge region-deleted variant genes (GOSTa:PrsA-sfGFP, GOSTa:PrsA DS-sfGFP, GOSTa:PrsA DH-sfGFP, and GOSTa:PrsA WD-sfGFP, respectively) and culturing the transformants.

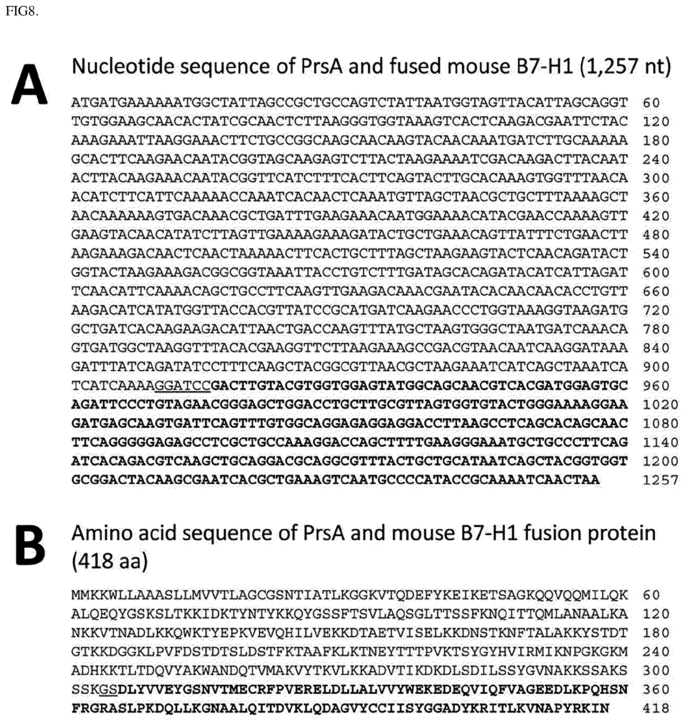

FIG. 8 shows the information of the nucleotide sequence (A) and amino acid sequence (B) of a protein encoded by the *PrsA* gene fused at the 3' end with the mouse *B7-H1* gene. The nucleotide and amino acid sequences of PrsA are shown in regular bold, and the sequences corresponding to the mouse B7-H1 are shown in bold.

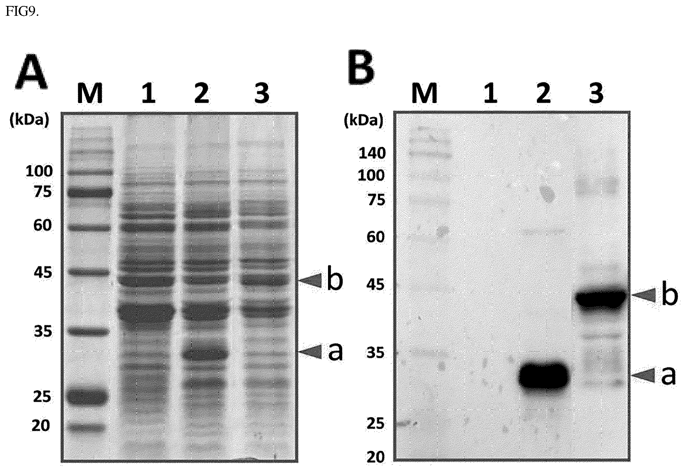

FIG. 9 shows the image (A) of a gel with the total protein stained with CBB after culturing *L. paracasei* transformed with a vector containing a fusion protein coding sequence fused to the mouse *B7-H1* gene at the 3' end of the *PrsA* gene, and the Western blot results (B) using anti-PrsA antibodies. M: protein size marker, 1: non-transformed *L. paracasei,* 2: GOSTa:PrsA-transformed *L. paracasei* control, 3: GOSTa:PrsA-mB7H1-transformed L. *paracasei* , a: location of PrsA protein size, b: location of PrsA-mB7H1 fusion protein size.

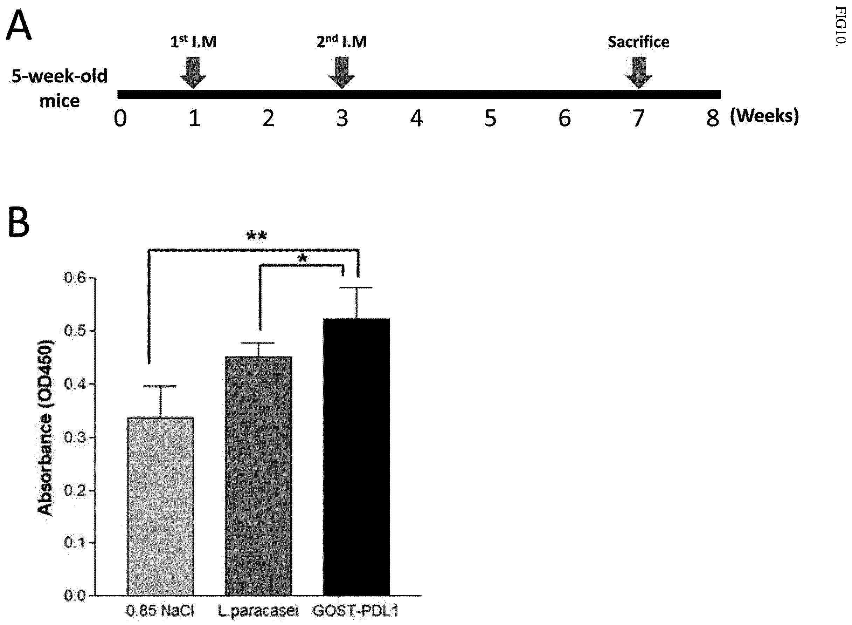

FIG. 10 shows the results of measuring an anti-B7H1 antibody level in serum four weeks after intramuscularly injecting dead cells of *L. paracasei* transformed with a GOSTa:PrsA-mB7H1 vector twice at two-week intervals into a mouse: (A) shows an animal experiment stage, indicating the timing of intramuscular injection and timing of serum collection, and (B) shows the result of ELISA assessing anti-B7H1 antibodies in six mice administered GOSTa:PrsA-mB7H1/*L*. *paracasei.* As a result of t-test analysis, * indicates p<0.05, and ** indicates significance at p<0.01.

### [Modes of the Invention]

To achieve the purpose of the present invention, the present invention provides a recombinant vector for the cell surface display of a target protein, in which a polynucleotide encoding PrsA consisting of the amino acid sequence of SEQ ID No. 2 or its variant and a gene encoding a target protein are sequentially linked downstream of a PrsA promoter consisting of the nucleotide sequence of SEQ ID No. 3.

The PrsA protein according to the present invention may consist of the amino acid sequence of SEQ ID No. 2 derived from *Lactobacillus sakei,* but the present invention is not limited thereto.

The scope of the PrsA according to the present invention includes a protein having an amino acid sequence represented by SEQ ID No. 2 and functional equivalents of the protein. The "functional equivalents" refer to proteins that have at least 30%, preferably, 40%, and more preferably, 50% sequence homology with the amino acid sequence represented by SEQ ID No. 2 as a result of addition, substitution, or deletion of amino acids, and exhibit substantially the same physiological activity as the protein represented by SEQ ID No. 2. The "substantially the same physiological activity" refers to the activity of displaying a target protein on the cell surface.

In addition, in the recombinant vector according to the present invention, the PrsA variant may be one in which residues 162 to 166 in the amino acid sequence of SEQ ID No. 2 are deleted, residues 281 to 303 in the amino acid sequence of SEQ ID No. 2 are deleted, or residues 162 to 166 and residues 281 to 303 in the amino acid sequence of SEQ ID No. 2 are deleted, but the present invention is not limited thereto.

In the present invention, residues 162 to 166 in the amino acid sequence of SEQ ID No. 2 is the hinge region of the PrsA protein, and residues 281 to 303 in the amino acid sequence of SEQ ID No. 2 is a serine-rich domain.

In one embodiment of the present invention, a polynucleotide encoding PrsA consisting of the amino acid sequence of SEQ ID No. 2 may consist of the nucleotide sequence of SEQ ID No. 1, but the present invention is not limited thereto. In addition, homologs of the above nucleotide sequence are included within the scope of the present invention. "% sequence homology" for a polynucleotide is determined by comparing two optimally aligned sequences with a comparative region, and a part of the polynucleotide sequence in the comparative region may have an addition or deletion (i.e., a gap) compared to the reference sequence (not including an addition or deletion) for the optimal alignment of the two sequences.

The term "recombinant cell" used herein refers to a cell that replicates a heterologous nucleic acid, expresses the nucleic acid, or expresses a peptide, a heterologous peptide or a protein encoded by a heterologous nucleic acid. A recombinant cell may express a gene or gene fragment that is not found in the natural form of the cell either in a sense or antisense form. In addition, a recombinant cell may express a gene found in the natural form of the cell, but the gene has been modified and reintroduced into a cell by an artificial means.

In addition, the term "vector" is used to refer to DNA fragment(s) and nucleic acid molecules, which are delivered into cells. A vector may replicate DNA and may be reproduced independently in host cells. The term "carrier" is often used interchangeably with "vector." The term "expression vector" means a recombinant DNA molecule containing a desired coding sequence, and an appropriate nucleic acid sequence essential for expressing the coding sequence operably linked thereto in a particular host organism.

In the present invention, a polynucleotide encoding PrsA or a variant thereof and a gene sequence encoding a target protein may be inserted into a recombinant expression vector. The term "recombinant expression vector" means a bacterial plasmid, a phage, a yeast plasmid, a plant cell virus, a mammalian cell virus, or other vectors. In general, any plasmid or vector may be used as long as it can replicate and stabilize within a host. The important characteristics of the expression vector are that it has a replication origin, a promoter, a marker gene, and a translation control element.

An expression vector including a polynucleotide encoding PrsA or its variant of the present invention, a gene sequence encoding a target protein, and an appropriate transcription/translation control signal may be constructed by a method known in the art. The method includes *in vitro* recombinant DNA technology, DNA synthesis technology, and *in vivo* recombination technology. The DNA sequence may be effectively linked to an appropriate promoter in an expression vector to drive mRNA synthesis. In addition, the expression vector may include a ribosome-binding site as an expression initiation site and a transcription terminator.

The term "polynucleotide" used herein refers to a polymer of nucleotides in which nucleotide units (monomers) are linked together in a long chain by covalent bonds, and a DNA or RNA strand of a certain length or longer, and more particularly, a polynucleotide fragment encoding the variant.

In addition, in a recombinant vector according to one embodiment of the present invention, the polynucleotide encoding PrsA or a variant thereof may be operably linked downstream of the PrsA promoter consisting of the nucleotide sequence of SEQ ID No. 3, but the present invention is not limited thereto.

The "operably linked" used herein means that one nucleic acid fragment is linked with another nucleic acid fragment such that its function or expression is affected by the other nucleic acid fragment. That is, the polynucleotide encoding PrsA or a variant thereof may be linked such that its expression can be controlled by the PrsA promoter consisting of the nucleotide sequence of SEQ ID No. 3.

The term "target protein" used herein refers to a protein that can be displayed on the cell surface of a microorganism transformed by inserting a polynucleotide encoding the protein into a recombinant vector by one of ordinary skill in the art.

In the recombinant vector according to the present invention, the target protein may be any one selected from the group consisting of a ligand protein, a receptor protein, an enzyme protein, and viral and bacterial proteins, but the present invention is not limited thereto. Proteins such as hormones, hormone analogs, enzyme inhibitors, antibodies or fragments thereof, toxin proteins, cytokines, transcription regulatory factors, or blood coagulation factors may also be included in the scope of the target protein according to the present invention.

The recombinant vector according to the present invention may allow a target protein to be displayed on a cell surface, and is an *E. coli-*lactic acid bacteria shuttle vector.

The present invention also provides a microorganism transformed with the recombinant vector for the cell surface display of a target protein.

In the present invention, the microorganism is preferably lactic acid bacteria, and more preferably, lactic acid bacteria of the genus *Lactobacillus,* but the present invention is not limited thereto.

The present invention also provides a method of displaying a target protein on the surface of a microorganism, which includes transforming a microorganism with a recombinant vector for the cell surface display of a target protein.

In the method of displaying a target protein on the surface of a microorganism according to the present invention, the recombinant vector for cell surface display, the target protein, and the microorganism are as described above.

A method of delivering the recombinant vector of the present invention into a microorganism, that is, a transforming method, may be implemented by a CaCl₂ method, a Hanahan method (Hanahan, D., 1983 J. Mol. Biol. 166, 557-580), conjugation (Heinze et al. BMC Microbiology 2018, 18:56), and electroporation, but the present invention is not limited thereto.

The present invention also provides a method of preparing a microorganism with a target protein displayed on the surface of a cell, which includes culturing a microorganism transformed with a recombinant vector for the cell surface display of a target protein to display the target protein on the cell surface, and recovering a microorganism with the target protein displayed on the cell surface prepared by the method.

In the method of preparing a microorganism with a target protein displayed on its cell surface according to the present invention, the recombinant vector for cell surface display, the target protein, and the microorganism are as described above.

In addition, the culturing of the transformed microorganism may be performed in a medium suitable for the production of a target protein using a known technique. A suitable culture medium may be obtained commercially or prepared according to the ingredients and composition ratio described in publications such as the catalog of the American Type Culture Collection, but the present invention is not limited thereto.

In the microorganism with a target protein displayed on its cell surface according to one embodiment of the present invention, the microorganism may be lactic acid bacteria that display B7-H1 (B7 homolog 1, also known as Programmed death-ligand 1 or cluster of differentiation 274) or a fragment thereof on the cell surface, but the present invention is not limited thereto. B7-H1 (or PD-L1) is a type of immune checkpoint inhibitor and is a major target in the development of immunotherapy drugs that activate immune cells to attack cancer cells.

The present invention provides an injectable or oral preparation, which includes a microorganism displaying B7-H1 or a fragment thereof as a target protein on the cell surface as an active ingredient using bacteria belonging to the genus *Lactobacillus,* which are GRAS microorganisms, as an antigen carrier.

The term "injectable preparation" used in the present invention refers to a preparation suitable for being injected into a human and/or a vertebrate, and the injection is intradermal, subcutaneous, intramuscular or intravenous injection. This preparation may be sterile, pyrogen-free, and have a physiologically acceptable pH. The pH of the injectable preparation is particularly important with regard to safety and comfort when injected, and especially when the preparation is supplied as a liquid preparation. A suitable preparation may contain a preservative, such as sodium benzoate, methylparaben, and propylparaben, and its pH may be pH 6.8 to 8.0 at 25 °C. The pH is preferably maintained by a buffer.

In addition, the oral preparation may be a powder, a granule, a tablet, a capsule, a trophy, a suspension, an emulsion, a syrup, or an aerosol. The solid preparation for oral administration may be formulated by mixing one or more excipients, for example, by mixing starch with calcium carbonate, sucrose, lactose, or gelatin. In addition to simple excipients, lubricants such as magnesium stearate and talc may also be used.

The present invention also provides a composition for inducing immunity in vertebrates other than humans, which includes the microorganism as an active ingredient.

The composition for inducing immunity according to the present invention includes lactic acid bacteria that display an antigen as a target protein on the cell surface as an active ingredient using a recombinant vector for the cell surface display of a target protein according to the present invention, and an immune response may be induced by administering the lactic acid bacteria to a vertebrate individual other than a human. The vertebrate is preferably a mammal other than a human, but the present invention is not limited thereto.

The composition for inducing immunity of the present invention contains lactic acid bacteria displaying an antigen on their cell surface, which are recognized as GRAS microorganisms. It may have an efficient and excellent immune-inducing effect by successfully inducing immunity through a general administration method, resulting in a disease treatment effect caused by the induction of immunity.

The present invention also provides a method of manufacturing a protein array, which includes immobilizing a microorganism, which is prepared by the method of preparing a microorganism of the present invention and has a target protein displayed on its surface, on a substrate surface.

The protein array provides a means, like a DNA array or DNA chip, in which various proteins, particularly, antibodies, are aligned on a solid surface to analyze whether or not a target protein is expressed in specific cells and the expression level of the protein. To manufacture the protein array, a protein to be aligned has to be secured, and the protein has to be immobilized on a solid surface. During the analysis process using a protein array, various treatments such as a high temperature, a salt concentration, and a pH change are performed to bind to the immobilized protein and wash away unbound proteins, and thus it is necessary to immobilize a stabilized protein that can withstand such harsh environments. However, cloning thousands to tens of thousands of protein genes into an expression vector, performing expression and isolation of the proteins, and immobilizing them on a solid surface requires a lot of repetitive tasks. Therefore, there is a need to make the above tasks simpler and faster.

For the process of manufacturing a protein array of the present invention, a manufacturing method conventionally used in the art may be applied. The protein array manufactured by the method of the present invention may be used for a diagnostic kit, gene expression analysis, analysis of interactions between proteins, or between proteins and ligands, and between antigens and antibodies, metabolic process analysis, exploration of novel enzymes or modified enzymes, combinatorial biochemical synthesis, and biosensors.

A solid substrate that can be used in the present invention includes glass (e.g., functional group-exposed glass), Si, Ge, GaAs, GaP, SiO, SiN₄, modified silicon nitrocellulose, polyvinylidene fluoride, polystyrene, polytetrafluoroethylene, polycarbonate, nylon, fiber, or a combination thereof. A linker molecule is attached to the substrate for protein immobilization, and it is preferable for the remaining portion that is not spotted to be blocked. Meanwhile, the amount of surface-displayed cells of the present invention applied to each spot (or address) is determined according to the array type. The interaction between the surface-displayed protein of the present invention immobilized on the solid substrate and a sample may be detected using the unique characteristics of the protein (e.g., immunoreactivity), or a labeling material suitable for the surface-displayed protein (e.g., a fluorescent material, a luminescent material, a radioactive material, or an epitope) to detect the signal change of the labeling material. The analysis of the final results obtained by the protein array of the present invention may be performed using an automated device known in the art as a "scanner" or "reader."

The present invention also provides a method of inducing immunity in a vertebrate, which includes administering a microorganism that is prepared by the method of preparing microorganisms of the present invention and has an antigen displayed on its surface to a vertebrate.

In the method of inducing immunity in a vertebrate according to the present invention, the vertebrate is preferably a mammal other than a human, but the present invention is not limited thereto.

Throughout the specification, when one part "includes" a component, it means that it may also include other components, not excluding components unless otherwise specifically stated. In addition, throughout the specification, the term "combination thereof" included in the Markush-type expression refers to a mixture or combination of one or more selected from the group consisting of constituents described in the Markush-type expression, that is, one or more selected from the group consisting of the components.

Hereinafter, the present invention will be described in detail with reference to the following examples. The following examples are merely provided to exemplify the present invention, and the contents of the present invention are not limited to the following examples.

### Example 1. Isolation and selection of lactic acid bacterial from traditional fermented kimchi

To isolate lactic acid bacteria, which are GRAS microorganisms, from gat-kimchi (Leaf Mustard kimchi) and cabbage kimchi manufactured by traditional methods in Changwon-si, Gyeongnam, 1 mL of kimchi broth was added to 100 mL of MRS (phosphate-yeast-peptone-sodium nitrate; 6 g/L K₂HPO₄, 2 g/L KH₂PO₄, 5 g/L Bacto-Trypton, 2.55 g/L NaNO₃, and 10 g/L yeast extract) liquid medium, and enrichment culture was performed under anaerobic conditions at 30 °C for 24 hours. Each obtained enrichment culture was diluted and spread on an MRS agar medium, and cultured for 48 hours at 30 °C under anaerobic conditions. The grown colonies were selected and subjected to 16S rDNA sequencing analysis. As a result, as shown in Table 1 below, various known microorganisms with a 16S rDNA sequence homology of 99.6% or more were isolated from each sample.

**[Table 1]**

| Result of 16S rDNA sequencing analysis of microorganisms isolated from gat-kimchi (Leaf Mustard kimchi) | | | |
|---|---|---|---|
| Sample # | Strain | Score(Bits) | Identities(%) |
| FT003 | *Lactobacillus paracasei* | 2563 | 1430/1435 (99.7%) |
| FT007 | *Lactobacillus sakei* | 2591 | 1405/1406 (99.8%) |
| FT016 | *Leuconostoc mesenteroides* | 2514 | 1361/1361 (100%) |
| FT026 | *Lactobacillus paracasei* | 2582 | 1398/1398 (100%) |
| FT028 | *Lactobacillus plantarum* | 2604 | 1444/1459 (99.6%) |

### Example 2. Screening of Lactobacillus sakei-overexpressed cell membrane protein

A strain used in an experiment (Sample #: FT007, *L. sakei*) was cultured in an MRS medium (Difco^{™} Lactobacilli MRS Broth, BD) under anaerobic conditions for 24 hours, washed with 0.85% saline three times, suspended in a 50 mM ammonium bicarbonate (NH₄HCO₃, pH 8.0) buffer, and treated with the lysyl endopeptidase (Lys-C, Cat# NC9223464, Wako) enzyme for 2 hours. Next, lactic acid bacteria was removed by centrifugation at 4,500 × g for 20 minutes, the peptide fragments degraded by Lys-C, which were derived from protein present in the outer membrane, contained in a buffer solution, which is a supernatant, were purified using a C18 SepPak cartridge (Sep-Pak C18 1 cc Vac, WAT054955, Waters), and the Lys-C peptide fragments recovered in an 80% acetonitrile solution were lyophilized at -80 °C for 24 hours. The dried peptide pool was dissolved in 20 µL of ammonium bicarbonate buffer, and 5 µL of the dissolved pool was used for C18 column-equipped nano_LC (EASY-nLC1000, Thermo Fisher) liquid chromatography to separate peptides, the separated and flowing solution was subjected to electrospray ionization and the sequence of each peptide was analyzed using a mass spectrophotometer (LTQ Orbitrap Velos LC-MS/MS, Thermo). The presence of the PrsA lipid membrane protein present in large quantities in the outer membrane was confirmed by sequentially separating the identified peptides using a C18 hydrophobic column with a concentration gradient of 2% to 30% acetonitrile and performing a MASCOT database search (Matrix Science) to identify the outer membrane proteins. FIG. 1 shows that, among the results of five Lys-C cleaved MS/MS analysis peptide sequences confirmed as PrsA lipid membrane proteins, the MS/MS result of the representative peptide sequence, amino acids 255 to 264 (N-WANDQTVMAK-C: SEQ ID No. 6).

### Example 3. Cloning of genes encoding L. sakei-derived PrsA promoter and PrsA protein

Based on the strain information and identified protein information obtained in Examples 1 and 2, the PrsA protein is assumed to be a membrane protein with high possibility of being an anchor protein for cell surface display, and since it is displayed in large quantities on the outer cell wall, the promoter inducing the expression of the PrsA protein can be assumed to be a very potent promoter. Therefore, the *L. sakei PrsA* gene information and the information of the promoter that regulates the synthesis of *PrsA* mRNA were confirmed in the KEGG database (https://www.kegg.jp). The sequence of 300 bases from the ATG start codon of the *PrsA* gene in the 5' upstream direction was considered as the PrsA promoter, and the chromosomal DNA of *L. sakei* isolated and purified using a primer set [5'-end promoter of PrsA of sFT007: 5'-aa act gca gga aat caa aac aac agc tg-3' (underline: *Pst* I recognition site, SEQ ID No. 7) and 3'-end of PrsA of sFT007: 5'-ttt-tct-ata-tta tta gga tcc ttt tga tga tga ttt gac-3' (underline: *Xba* I recognition site, SEQ ID No. 8)] as a template to amplify the 1.22 kb gene by PCR, and then the lactic acid bacteria-*E*. *coli* shuttle vector pFT003 (Korean Patent Publication No. 10-0469800, the same as pHCE1LB:BCA) was cloned using *Pst* I and *Xba* I, thereby preparing pGOSTa:PrsA (FIG. 3).

### Example 4. Induction of PrsA expression on outer cell membrane of Lactobacillus paracasei

Lactic acid bacterial colonies transformed by introducing the pGOSTa:PrsA prepared in Example 3 to the *L. paracasei* FT003 strains shown in Table 1 by electroporation were inoculated into 15 mL of MRS liquid medium and cultured at 30 °C for 24 hours under anaerobic conditions. 5 mL of the culture was collected and washed with 0.85% saline three times, the cells were disrupted using a bead beater and recovered, and 20 µg of the recovered total protein was electrophoresed on a 12.5% SDS-PAGE gel and stained by Coomassie brilliant blue (CBB) staining (FIG. 4A). In addition, the protein bands separated by size in the SDS-PAGE were transferred to a PVDF membrane and Western blotting was performed using anti-PrsA antibodies. As a result, the exact location and size of the PrsA protein was able to be confirmed, and the expression level of the PrsA protein in the total protein was able to be confirmed (FIG. 4B).

Generally, it is not common to find a method that can stably express a target protein in large quantities through genetic manipulation in lactic acid bacteria. However, as shown in FIG. 4, in *L. paracasei* host cells, the *L. sakei-derived* PrsA promoter can stably express *L*. sakei-derived PrsA in large quantities without competing with the PrsA promoter present on the *L. paracasei* chromosome, as confirmed by CBB staining and Western blotting using PrsA-specific antibody (FIGS. 4A and 4B). Particularly, it was confirmed that the anti-PrsA polyclonal antibody (KOMA#24462) produced using the purified *L. sakei* PrsA protein does not cross-react with *L. paracasei-derived* PrsA (amino acid sequence homology between *L*. *sakei-* and *L. paracasei-derived* PrsA proteins: 54%, amino acid sequence similarity between *L*. *sakei-* and *L. paracasei-derived* PrsA proteins: 71%), and it can be confirmed that the *L. sakei-*derived PrsA protein biosynthesized in the pGOSTa:PrsA surface display system accounts for 3% or more of the total protein expressed in *L. paracasei.*

Next, to confirm whether the *L. sakei-derived* PrsA protein, which was confirmed to be expressed in large quantities, is present on the cell membrane of *L*. *paracasei,* 0.5 mL of a solution containing 1.5 mg/mL of the total protein disrupted using a bead beater was centrifuged at 4 °C for 4 hours at a high speed of 20,000 × g, the supernatant was transferred to a new tube, and the pellet was re-dissolved in 0.5 mL of PBS. 8 µL of each protein solution containing the total protein was taken and mixed with 2 µL of a 5X SDS-PAGE sample buffer, thermally treated at 97 °C for 5 minutes, and subjected to electrophoresis on a 10% SDS-PAGE gel to perform CBB staining. As a result, as confirmed in FIG. 4C, the 31.4 kDa PrsA protein derived from *L. sakei,* present in the total protein sample in lane 1, was not found in the cytoplasmic solution in lane 2, but found entirely in the cell membrane protein solution in lane 3. Therefore, it was able to confirmed that most of the PrsA protein displayed in large quantities in the GOSTa:PrsA vector is present on the cell membrane of *L. paracasei.*

### Example 5. Manufacture of PrsA-sfGFP fusion protein expression vector (pGOSTa:PrsA-sfGFP)

An *sfGFP* gene cloned into a pQI30 vector was amplified by PCR using the forward primer sfGFP *BamH I* [5'-aaa agg atc cat gag caa agg aga ag-3' (underline: *BamHI* recognition site, SEQ ID No. 9)] and the reverse primer sfGFP *Kpn* | [5'-tct tgg tac ctt tgt aga gct cat cca-3' (underline: *Kpn* | recognition site, SEQ ID No. 10)]. Afterward, the resulting genes were treated with *BamH I* and *Kpn* I, and the pGOSTa:PrsA vector manufactured in Example 3 was treated with the same restriction enzyme, and then the two DNA fragments were ligated to manufacture pGOSTa:PrsA-sfGFP (FIG. 5).

### Example 6. PrsA structural analysis, modification of PrsA anchoring motif, and construction of sfGFP-fused surface display system

The PrsA protein has a structure in which amino acid 21 at the N-terminus, cysteine, is covalently linked to carbon in the glycerol molecule of a lipid, which is the main component of the cell membrane, and is tightly anchored to the cell membrane, and the C-terminus of PrsA consists of an NC domain located close to the N-terminus. In addition, the PrsA protein has a homodimer structure in which the N-terminus and C-terminus of each monomer are close to each other and is formed in a cyclic shape. In addition, the serine-rich domain present at the C-terminus of each PrsA is determined to play an important role in the formation of the NC domain structure. It is presumed that the serine-rich domain interacts with the peptidoglycan layer, which is the cell wall component of Gram-positive bacteria, and plays a role in the maintenance of structural stability within the cell wall.

The key to display on the lactic acid bacterial cells is to maximize the display of specific proteins outside the outer membrane rather than inside the peptidoglycan layer. The present inventors determined that a foreign protein would be sent outside the cell wall much more efficiently than the wild-type PrsA by deleting 23 amino acid residues (DLSDILSSYGVNAKKSSAKSSSK, SEQ ID No. 4, FIG. 2) of the serine-rich domain of PrsA. To remove the serine-rich domain of the PrsA protein, PCR was performed using the pGOSTa:PrsA vector manufactured in Example 3 as a template and the 5'-end promoter of PrsA of the sFT007 primer (SEQ ID No. 7) and the delete C-term serine-rich domain of PrsA primer [5'-ggg ggg tac ctt atc agg atc cTT TAT CCT TGA TTG TTA CGT CGG C-3' (underline: *BamH* I recognition site, SEQ ID No. 11)], and the amplified 1.4 kb DNA fragment was treated with restriction enzymes *Pst* I and *BamH I* and recovered. The restriction enzyme-treated DNA fragment and the 7.25 kb vector DNA fragment recovered after treating the pGOSTa:PrsA-sfGFP manufactured in Example 5 with the same restriction enzymes *Pst* I and *BamH* I were ligated to obtain pGOSTa:PrsA DS-sfGFP.

As previously described about the PrsA structure, PrsA is known to have a curved structure with the N-terminus and the C-terminus close together. In the present invention, by deleting the 'KDNST' amino acid sequence (SEQ ID No. 5, FIG. 2), which is located in the middle position of the PrsA protein sequence and assumed to play the most critical role in the hinge region, after confirming the hinge region through the structural analysis, it was determined that a specific protein fused to PrsA can be efficiently sent to the outside of the cell wall. To delete the five amino acids in the hinge region, PCR was performed using the pGOSTa:PrsA-sfGFP vector obtained in Example 5 as a template and the hinge deletion forward primer [5'-ccc cct cga gaa gaa gta ctc aac aga t-3' (underline: *Xho* I recognition site, SEQ ID No. 12)] and the hinge deletion reverse primer [5'-ccc cct cga gct taa gtt cag aaa taa c-3' (underline: *Xho* I recognition site, SEQ ID No. 13)], and the 8.45 kb PCR product was recovered. The collected PCR product was treated with the restriction enzyme *Xho* I and then self-ligated to obtain pGOSTa:PrsA DH-sfGFP.

The PrsA WD variant from which both the PrsA serine-rich domain and the hinge region were deleted was subjected to PCR using a pGOSTa:PrsADS-sfGFP plasmid as a template, the hinge deletion full sequence forward primer [5'-ccc cct cga gaa gaa gta ctc aac aga t-3' (underline: *Xho* I recognition site, SEQ ID No. 14)], and the hinge deletion full sequence reverse primer[5'-ccc cct cga gct taa gtt cag aaa taa c-3' (underline: *Xho* I recognition site, SEQ ID No. 15)]. The amplified 7.27 kb DNA fragment was self-ligated after *Xho* I treatment to obtain pGOSTa:PrsA WD-sfGFP.

### Example 7. Confirmation of E. coli cell surface display of sfGFP fused with PrsA modified anchoring motif

Four types of the obtained plasmids, pGOSTa:PrsA-sfGFP, pGOSTa:PrsA DS-sfGFP, pGOSTa:PrsA DH-sfGFP, and pGOSTa:PrsA WD-sfGFP were introduced into the *L. paracasei* isolated from gat-kimchi (Leaf Mustard kimchi) by electroporation, and each of four types of the transformed *L. paracasei* strains were cultured in an MRS liquid medium under anaerobic conditions for 24 hours. After collecting 5 mL of four types of *L. paracasei* cultures, each cell culture was washed with 0.85% saline three times and disrupted with a bead beater to collect total proteins. The total protein of each *L. paracasei* was quantified by a bicinchoninic acid (BCA) method, and 20 µg of the total protein was subjected to electrophoresis on two sheets of 12.5% SDS-PAGE gel prepared in advance to separate proteins by size. After electrophoresis, one PAGE gel was stained with CBB, and the other gel was transferred to a PVDF membrane and subjected to Western blotting using an anti-sfGFP antibody (Santa Cruz Biotechnology, Cat# sc-9996).

As a result, as shown in FIG. 6, CBB images show that the control and five sample proteins including *L*. *paracasei* are evenly distributed in SDS-PAGE, and the Western blotting image confirmed that sfGFPs fused with PrsA and PrsA variants were stably expressed in *L*. *paracasei.* In addition, each fused protein shows a slight difference in the theoretical molecular weight at the same position for each mutant, and shows a slight difference in surface display level in the following order of a PrsA hinge region-deleted anchor, a PrsA hinge region and serine-rich domain-deleted anchor, a PrsA serin-rich domain-deleted anchor as compared to the wild-type PrsA anchor protein. Above, it was confirmed that the target protein sfGFP in four types of plasmids having PrsA and PrsA-modified anchoring motifs, such as pGOSTa:PrsA-sfGFP, pGOSTa:PrsA DS-sfGFP, pGOSTa:PrsA DH-sfGFP, and pGOSTa:PrsA WD-sfGFP was stably expressed in lactic acid bacteria.

Next, whole-cell ELISA (Bonnie L. Elder et al., J. Clin. Microbiol. 1982, 16:141-144; Albritton et al., PLOS One 2017, 12(8):e0183101) was used to compare and analyze whether, in the four types of plasmids, sfGFP expressed by fusion with PrsA or PrsA-modified anchoring motifs was effectively displayed outside the cell surface of *L. paracasei.*

As a result, as confirmed in FIG. 7, the levels of sfGFP displayed on the cell surface were confirmed in the following order of the PrsA hinge region-deleted anchor (PrsA DH), PrsA hinge region and serine-rich domain-deleted anchor (PrsA WD), PrsA serine-rich domain-deleted anchor (PrsA DS) and wild-type PrsA anchor (PrsA) motifs. As a result, it was found that the wild-type PrsA and PrsA-modified anchoring motifs effectively display the target protein on the cell surface of lactic acid bacteria, and the PrsA-modified anchoring motif has a better effect on the cell surface display of the target protein than the wild-type PrsA.

### Example 8. Preparation of surface display vector pGOSTa:PrsA-mB7-H1 and surface display

The mouse *B7-H3* gene was amplified by PCR using PD-L1 (CD274) (NM_021893) Mouse Tagged ORF clone (ORiGENE, Cat # MR203953) containing the mouse *B7-H1* gene as a template, the mouse B7-H1 forward primer [5'-AAA GGA TCC GAC TTG TAC GTG GTG GAG-3' (underline: *BamH* I recognition site, SEQ ID No. 16)], and the mouse B7-H1 reverse primer [5'-GGG TCT AGA ACT AGT GTC GAC TTA GTT GAT TTT GCG GTA TGG GGC ATT-3' (underline: *Xba* I recognition site, SEQ ID No. 17)], treated with the restriction enzymes *BamH* I and *Xba* I to collect DNA fragments of approximately 360 bp. The collected 360 bp DNA fragment and the vector manufactured in Example 3 were treated with *BamH I* and *Xba* I, thereby collecting a 360 bp DNA fragment and a 7.7 kb DNA fragment, respectively, and these fragments were ligated to manufacture pGOSTa:PrsA-mB7-H1. The nucleotide sequence of the entire gene encoding the PrsA-mB7-H1 fusion protein and the amino acid sequence of the fused protein are shown in FIG. 8.

The obtained pGOSTa:PrsA-mB7-H1 plasmid was introduced into the *L. paracasei* FT003 strain isolated in Example 1 by electroporation, and the transformed lactic acid bacteria colonies were cultured by the method described in Example 4. After collecting 5 mL of the culture, it was washed with 0.85% saline, disrupted using a bead beater, and 20 µg of the total protein collected thereby was subjected to electrophoresis on a 12.5% SDS-PAGE gel and stained with CBB (FIG. 9A). In addition, protein bands separated by size in the SDS-PAGE were transferred to a PVDF membrane, and subjected to Western blotting using an anti-B7-H1 antibody (R&D Systems Cat #AF1019) (FIG. 9B). As a result, the large-quantity expression and size of the PrsA-mB7-H1 fusion protein were able to be confirmed. In addition, the expression level of the PrsA-mB7-H1 fusion protein in the total protein was able to be confirmed, and it was confirmed that the PrsA-mB7-H1 fusion protein was most stably expressed in large quantities as confirmed by SDS-PAGE CBB staining.

### Example 9. Antibody inducing effect of lactic acid bacteria surface displaying mouse B7-H1 protein

To investigate the surface protein antigenicity of a *L. paracasei* FT003 strain transformed with the pGOSTa:PrsA-mB7-H1 manufactured in Example 8, the formation of neutralizing antibodies was determined. After the *L. paracasei* with an antigen displayed on its surface was treated with ethanol to kill the bacteria, the dead cells were washed with 0.85% sodium chloride solution three times, and 1 × 10⁷ cells of the dead cells were administered intramuscularly twice at two-week intervals to each of six 6-week-old male BALB/c mice, which had been housed at 5 weeks of age and acclimated for 1 week. Each of the 0.85% sodium chloride group as the control and the *L. paracasei* lactic acid bacteria-only experimental group included six mice. Serum from each experimental group was collected 4 weeks after the second intramuscular injection, and ELISA was used to measure and compare neutralizing antibody titers for each antigen. As a result, as shown in FIG. 10B, compared with the group administered 0.85% sodium chloride as a control and the group administered only a *L. paracasei* FT003 lactic acid bacteria carrier, the anti-B7-H1 antibody titer of the group that was administered the *L. paracasei* transformed with pGOSTa:PrsA-mB7-H1 was higher, which was statistically significant.

From the above results, it can be seen that the pGOSTa:PrsA vector of the present invention can be utilized as a platform technology that can display various target proteins on the cell surface of a microorganism using PrsA as a cell membrane anchor, and it can be confirmed that, when microorganism cells transformed with 'pGOSTa:PrsA-antigen' were administered to an animal for the purpose of utilizing a target protein displayed on a surface, antibodies against the antigen can be induced.

## Claims

1. A recombinant vector for the cell surface display of a target protein in which a polynucleotide encoding PrsA consisting of the amino acid sequence of SEQ ID No. 2 or its variant and a gene encoding a target protein are sequentially linked downstream of a PrsA promoter consisting of the nucleotide sequence of SEQ ID No. 3.

2. The recombinant vector of claim 1, wherein, in the variant of PrsA, residues 162 to 166 of the amino acid sequence of SEQ ID No. 2 are deleted.

3. The recombinant vector of claim 1, wherein, in the variant of PrsA, residues 281 to 303 of the amino acid sequence of SEQ ID No. 2 are deleted.

4. The recombinant vector of claim 1, wherein, in the variant of PrsA, residues 162 to 166 and residues 281 to 303 of the amino acid sequence of SEQ ID No. 2 are deleted.

5. The recombinant vector of claim 1, wherein the target protein is any one selected from the group consisting of a ligand protein, a receptor protein, an enzyme protein, and viral and bacterial proteins.

6. A microorganism transformed with the recombinant vector of any one of claims 1 to 5.

7. The microorganism of claim 6, which is a lactic acid bacterium.

8. A method of displaying a target protein on the surface of a microorganism, comprising:
transforming a microorganism with the recombinant vector of any one of claims 1 to 5.

9. A method of preparing a microorganism with a target protein displayed on its cell surface, comprising:
culturing the transformed microorganism of claim 6 to display a target protein on the cell surface; and
harvesting the microorganism with the target protein displayed on its cell surface.

10. The method of claim 9, wherein the target protein is any one selected from the group consisting of a ligand protein, a receptor protein, an enzyme protein, and viral and bacterial proteins.

11. A microorganism with a target protein displayed on its cell surface prepared by the method of claim 9.

12. The microorganism of claim 11, wherein the target protein is B7 homolog 1 (B7-H1) or a fragment thereof.

13. An injectable preparation comprising the microorganism of claim 12 as an active ingredient.

14. An oral preparation comprising the microorganism of claim 12 as an active ingredient.

15. A method of manufacturing a protein array, comprising:
immobilizing a microorganism, which is prepared by the method of claim 9 and has a target protein displayed on its surface, on a substrate surface.

16. A method of inducing immunity in a vertebrate, comprising:
administering a microorganism, which is prepared by the method of claim 9 and has an antigen displayed on its surface, to a vertebrate.
